# EUROPEAN PATENT SPECIFICATION

(11) **EP 1 666 086 B1**
(45) Date of publication and mention of the grant of the patent: **30.04.2008**
(21) Application number: 05257492.8
(22) Date of filing: 06.12.2005
(51) Int. Cl.: A61N 1/05

(54) **Automatic capture pacing lead**
Stimulationselektrode zur automatischen Fangschwellenbestimmung
Electrode de stimulation pour la détermination automatique du seuil de la capture

(30) Priority: 06.12.2004 US 5929
(43) Date of publication of application: 07.06.2006
(73) Proprietor: PACESETTER, INC., Sylmar, CA 91342-9221 (US)
(72) Inventor: Kroll, Mark W., Orono, MN 55391 (US); Jenney, Christopher R., Valencia, CA 91354 (US); Levine, Paul A., Santa Clarita, CA 91321 (US)
(74) Representative: Rees, David Christopher

(56) References cited:
- US-A- 5 314 459
- US-A- 5 376 206
- US-A- 5 398 683
- US-A- 5 824 028
- US-B1- 6 357 447

## Description

The present invention relates generally to electromedical devices and, more particularly, to implantable transvenous leads for electrically stimulating the tissue of the heart and for sensing the electrical potentials generated thereby.

Body implantable, transvenous leads may form the electrical connection between an implantable medical device such as a cardiac pacemaker and/or ICD and the heart tissue that is to be stimulated. Such systems may include an automatic capture pacing system such as the AutoCapture^{™} cardiac pacing system manufactured by St. Jude Medical, Inc., that incorporates, among other features, threshold-tracking algorithms including dynamic "beat-by-beat" capture confirmation to ensure capture at all times. In cardiac pacing, the "threshold" is defined as the minimum electrical energy or current required to cause cardiac muscle depolarization. The capture threshold can be reported as the minimum pulse amplitude (voltage or current), pulse duration, charge, energy or current density that results in consistent capture. In the clinical realm, the capture threshold is usually defined by the adjustable or programmable parameters of pulse amplitude (voltage) or pulse duration (milliseconds) or a combination of both. Hence, the capture threshold is the lowest voltage and/or pulse duration that results in consistent electrical activation or depolarization of the heart chamber to which the pacing stimulus is applied. Capture is commonly followed by mechanical contraction of the depolarized chamber. In the AutoCapture™ pacing system, every paced heart beat is monitored for the presence of an evoked response (the signal resulting from the electrical activation of the myocardium by a pacemaker) and if there is no evidence of capture, a higher output back-up pulse is delivered to assure effective capture. Pacing thresholds are regularly measured to determine the output energy level requirement, and the pacemaker's output level is regulated so as to be set just above the measured threshold, ensuring the lowest energy level required for capture thereby optimizing device longevity. If the threshold rises such that it exceeds the automatically set output, back-up pulses are delivered and the system will reassess the capture threshold and automatically reprogram the output setting. In the absence of such a tracking and continuous capture verification algorithm, the physician must program a safety margin of, for example, two to three times the measured capture threshold so as to protect the patient in case of changing energy requirements due to metabolic shifts, progression of disease and so forth that may occur between scheduled office evaluations. If the capture threshold is very stable, this results in a waste of energy and accelerates battery depletion. If the capture threshold experiences an excessive increase, the patient may not be protected and experience symptoms associated with loss of capture.

Presently, automatic capture pacing is accomplished by using unipolar or tip pacing (tip electrode-to-pulse generator case) and bipolar sensing between the tip and ring electrodes. Bipolar pacing is typically avoided because in this mode there is a tendency for the ring electrode to pace first, that is, there is a tendency to pace anodally from the ring electrode and in the original algorithm. This was difficult for the implanted system to detect. This results in a completely different morphology and polarity from tip pacing. Therefore, bipolar pacing typically has not been used for automatic capture pacing unless unipolar sensing is employed. Unipolar sensing, however, has a number of problems including the sensing of physiologically inappropriate signals such as myopotentials, that is, electrical signals that may originate in skeletal muscles in close proximity to the implanted pulse generator and may be interpreted as cardiac depolarizations resulting in inappropriate inhibition or triggering of a stimulating pulse. In addition, unipolar pacing (to allow bipolar sensing) can be a problem with ICD compromising its ability to recognize the low amplitude signals associated with ventricular fibrillation.

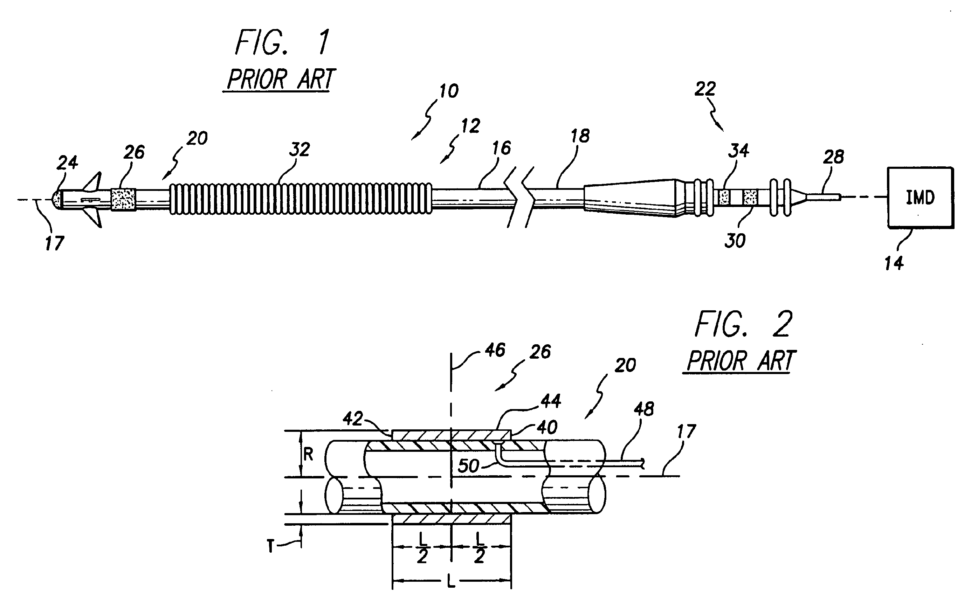

**FIGS. 1** and **2** show an example of a conventional, bipolar, transvenous pacing, sensing and defibrillating system 10 comprising a lead 12 and an implantable medical device (IMD) 14 that may comprise a pacemaker/ICD. The lead 12 includes a lead body 16 extending along a longitudinal central axis 17 and having a proximal end 18 and a distal end portion 20. The proximal end 18 of the lead body 16 incorporates a connector assembly 22 for connecting the lead body to the IMD 14.

The distal end portion 20 of the lead body 16 carries a tip electrode 24 and a ring electrode 26 proximally of the tip electrode. The tip and ring electrodes are coupled to corresponding terminal contacts 28 and 30, respectively, on the connector assembly 22 by means of electrical conductors enclosed within the lead body 16. The distal end portion 20 of the lead body 16 also carries a cardioverting and/or defibrillating electrode 32 electrically connected to a terminal contact 34 by means of a conductor within the lead body 16.

**FIG. 2** shows, in schematic form, the conventional ring electrode 26 carried by the distal end portion 20 of the lead body 16 shown in **FIG. 1****.** The ring electrode 26 has proximal and distal edges 40 and 42, respectively, and an outer cylindrical surface 44 having a radius R. The ring electrode 26 has an overall length, L, extending in the longitudinal direction of the lead, and a uniform thickness, T, along the entire length of the electrode. The ring electrode 26 is symmetrical about a transverse plane 46 equidistant from the proximal and distal edges 40 and 42. An electrical conductor 48 having a distal end 50 electrically connected to the ring electrode in conventional fashion connects the electrode to the terminal contact 30 on the connector assembly 22. By way of example, the conventional ring electrode of **FIG. 2** may have a length, L, of 1.0 cm, a radius, R, of 0.15 cm, and a thickness, T, of 0.1 mm.

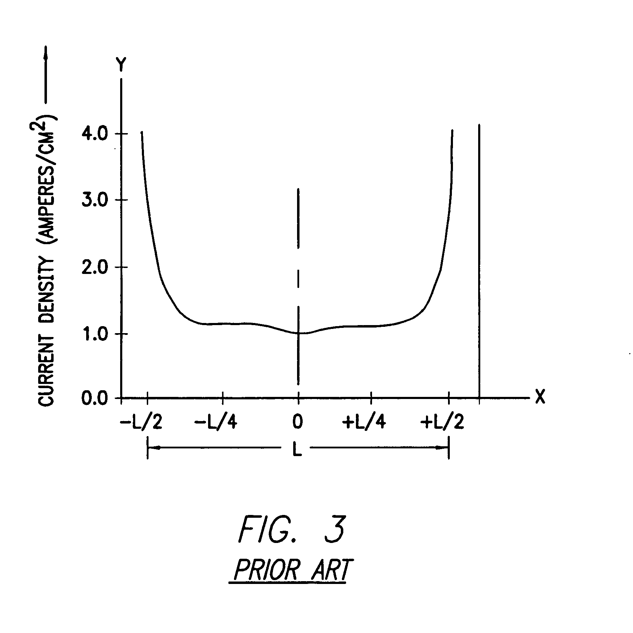

**FIG. 3****,** comprising a plot of current density (in amperes per cm²) as a function of distance along the length of the ring electrode 26, illustrates the problem of conventional ring electrodes that gives rise to anodal ring pacing. The current density plot of **FIG. 3** (which is derived from a solution of the field equations) is based on a 1.0 cm long ring electrode with the horizontal or X axis being the distance from the center (0) of the electrode to its ends (-L/2 and +L/2) and the vertical or Y axis being the current density in amperes per cm². It will be seen that the current density assumes a substantially constant, low value in the center portion of the ring but rises rapidly to essentially an infinite value at each of the edges of the ring. This extremely high current density along each edge of the ring will typically result in anodal ring pacing if the ring is in contact or otherwise in electrical communication with viable body tissue. The high current densities at the edges of the ring electrode, also known as "edge effects" or "hot spots", besides causing anodal ring pacing, can cause blood coagulation as well as damage to healthy tissue surrounding the targeted tissue. The fundamental current density vs. length characteristic of the plot shown in **FIG. 3** is equally applicable to large and small ring electrodes.
US Patent No. 5314459 discloses an electrode which has three discrete segments, the middle of which has a lower impedance than the outer two. The invention is defined in claim 1.

Preferably, a tip electrode is disposed at a distal extremity of the distal end portion of the lead body, the tip electrode being electrically coupled to a first terminal contact on a connector assembly attached to the proximal end of the lead body. Preferably, the ring electrode positioned along the distal end portion of the lead body proximally of the tip electrode, the ring electrode being electrically coupled to a second terminal contact on the connector assembly, the ring electrode having distal and proximal ends.

Preferably, an implantable cardiac pacing system comprising a pulse generator and a bipolar pacing lead as described.

Preferably, a method of fabricating an electrically conductive ring electrode for a pacing lead comprises forming adjacent each of the opposed ends of the ring electrode a region having an electrical resistance that is greater than that of the portion of the ring electrode between said regions.

The reduction of the current density at the higher resistance ends or end regions of the ring electrode increases the magnitude of the current that must be delivered to the ring electrode in order for it to pace anodally, thereby eliminating "hot spots" and inhibiting the tendency to pace anodally.

The invention may be carried into practice in various ways and some embodiments will now be described by way of example with reference to the accompanying drawings, wherein:

**FIG. 1** is a side view of a conventional bipolar transvenous pacing and defibrillation lead;

**FIG. 2** is a side view, partly in cross-section, of a portion of the lead of FIG. 1 showing details of a ring electrode carried by the lead;

**FIG. 3** is a plot of electrical current density as a function of distance along the length of the conventional ring electrode shown in **FIG. 2****;**

**FIG. 4** is side view of a bipolar transvenous pacing and defibrillation lead in accordance with one specific, exemplary embodiment of the invention;

**FIG. 5** is a side view, in cross-section, of a sensing ring electrode that may be carried by the lead of **FIG. 4****,** along with associated plots of surface resistivity and current density as a function of distance along the length of the electrode;

**FIG. 6** is a side view, in cross-section, of an alternative form of a sensing ring electrode that may be carried by the lead of **FIG. 4****;**

**FIG. 7** is a side view, in cross-section, of another form of a sensing ring electrode shown that may be carried by the lead of **FIG. 4****;** and

**FIG. 8** is side view of a multipolar transvenous pacing and defibrillation lead in accordance with an alternative embodiment of the invention.

Although the invention will be described in the context of implantable cardiac stimulation and sensing leads, it will be evident to those skilled in the art that the invention described herein has broader utility, being applicable to a wide variety of implantable medical leads for stimulating selected body tissue and sensing the electrical activity of such tissue. Further, although the invention is described herein in the context of a ring sensing electrode, it will be evident that the invention is applicable to a wide range of electrodes, including, without limitation, pacing and/or sensing electrodes and cardioverting/defibrillating electrodes, whether wound around a lead body or otherwise configured.

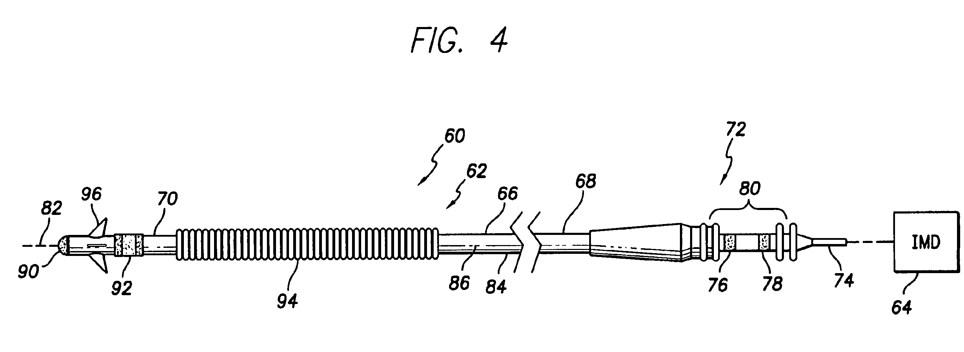

**FIG. 4** shows a transvenous pacing, sensing and defibrillating system 60 in accordance with one specific embodiment of the invention comprising a lead 62 and an implantable medical device (IMD) 64 that may comprise a pacemaker/ICD. The lead 62 includes a lead body 66 having a proximal end 68 and a distal end portion 70. The proximal end 68 of the lead body 66 incorporates a coaxial connector assembly 72 that may be compatible with a standard such as the proposed IS-4 standard for connecting the lead body 66 to the IMD 64. In the example shown in **FIG. 4****,** the connector assembly 72 includes a tubular pin terminal contact 74 and two annular terminal contacts 76 and 78 electrically coupled to electrodes along the distal end portion of the lead body. The connector assembly 72 is received within a receptacle (not shown) in the IMD 64 containing electrical terminals positioned to engage the terminal contacts 74, 76 and 78 on the connector assembly. As is well known in the art, to prevent ingress of body fluids into the receptacle, the connector assembly 72 may be provided with spaced sets of seals 80. In accordance with standard implantation techniques, a stylet or guide wire (not shown) for delivering and steering the distal end portion of the lead body 66 during implantation is inserted into a lumen of the lead body through the tubular pin terminal contact 74.

The lead body 66 extends along a central, longitudinal axis 82 and preferably comprises a tubular sheath or housing 84 made of an insulating, biocompatible, biostable polymer, for example, silicone rubber, polyurethane, or other suitable polymer and having an outer surface 86. Although various insulating housing materials are intended to be encompassed by the invention, silicone rubber is often preferred because of its flexibility and long term biostability.

The distal end portion 70 of the lead body 66 may carry one or more electrodes whose configurations, functions and placement along the length of the distal end portion will be dictated by the indicated stimulation therapy, the peculiarities of the patient's anatomy, and so forth. The lead body 66 illustrates but one example of the various combinations of stimulating and/or sensing electrodes that may be utilized. The distal end portion 70 of the lead body carries a tip electrode 90 and a ring electrode 92 proximally of the tip electrode. The tip and ring electrodes 90 and 92 are coupled to corresponding terminal contacts 74 and 76, respectively, on the connector assembly 72 by means of electrical conductors (not shown) within the housing 84. The distal end portion of the lead body also carries a cardioverting and/or defibrillating electrode 94 electrically connected to the terminal contact 78 by means of a separate electrical conductor (not shown) within the housing 84.

In conventional fashion, the distal end portion 70 of the lead body 66 may include passive fixation means 96 that may take the form of projecting tines for anchoring the lead body within a chamber of the heart. Alternatively or in addition thereto, the passive fixation or anchoring means may comprise one or more preformed humps, spirals, S-shaped bends, or other configurations manufactured into the distal end portion 70 of the lead body where the lead is intended for left heart placement within a vessel of the coronary sinus region. The fixation means may also comprise an active fixation mechanism such as a helix. It will be evident to those skilled in the art that any combination of the foregoing fixation or anchoring means may be employed.

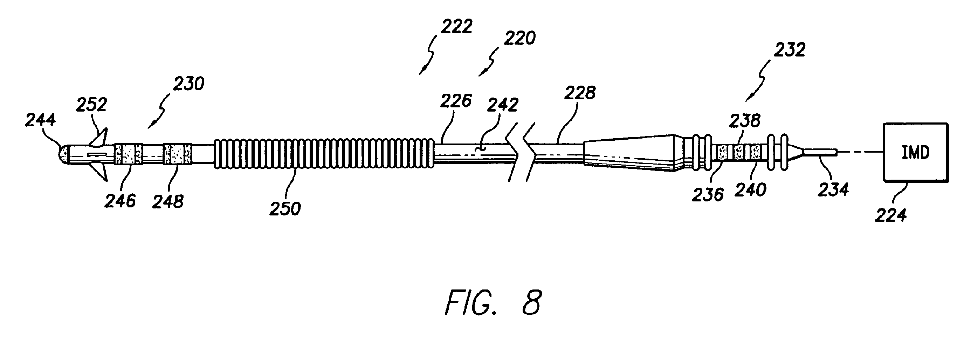

Other electrode arrangements may, of course, be utilized pursuant to lead constructions well known in the art. For example, an alternative electrode arrangement may include additional ring stimulation and/or sensing electrodes (see, for example, **FIG. 8** and the related description, below) as well as additional cardioverting and/or defibrillating coils spaced apart along the distal end of the lead body. Thus, as emphasized, **FIG. 4** is illustrative only; the distal end portion 70 of the lead body 66 may, for example, carry only cardioverting/defibrillating electrodes or a combination of pacing, sensing and cardioverting/defibrillating electrodes. The defibrillating electrodes are preferably of coil design, as shown, and for greater lead flexibility may comprise spaced apart, relatively short coils; alternatively, these electrodes may be made of an electrically conductive polymer.

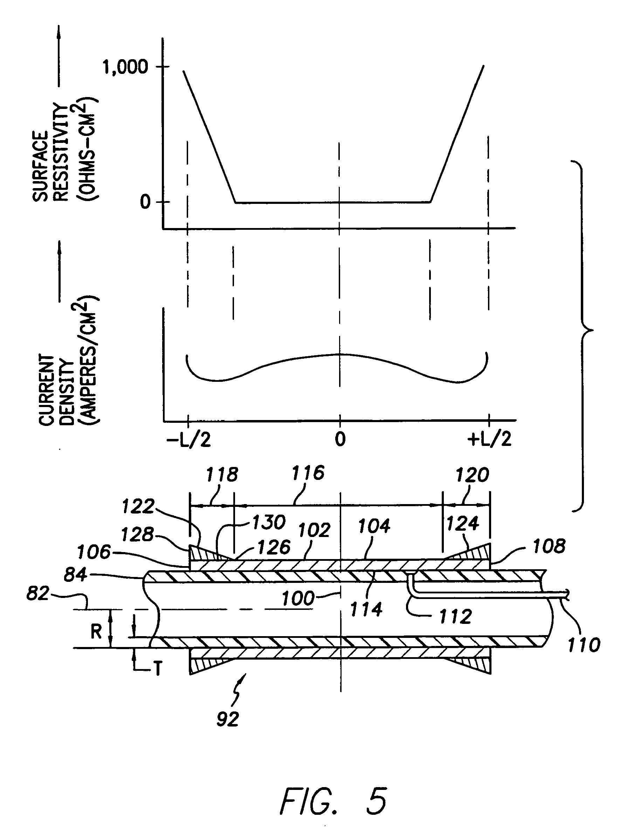

**FIG. 5** shows in greater detail the ring electrode 92 along with associated plots of surface resistivity, in ohms-cm², and current density, in amperes per cm², as a function of distance along the length of the electrode, using the center of the electrode, lying in a transverse central plane 100, as the origin (0). The ring electrode 92 has a main, annular body 102 having an outer cylindrical surface 104 and opposed distal and proximal ends 106 and 108, respectively. By way of example only, the ring electrode body 102 may have a length, L, of 1.0 cm, a radius, R, of 0.15 cm, and a thickness, T, of 0.1 mm. The electrode body 102 may be fabricated of, for example, MP35N alloy, a platinum/iridium alloy, stainless steel, or titanium, preferably coated with an agent such as titanium nitride, iridium oxide, platinum black, or the like.

The ring electrode 92 is connected to the terminal contact 76 on the connector assembly by means of an electrical conductor 110 having a distal extremity 112 electrically connected, for example, by a laser weld or crimping, to a central portion of an inner surface 114 of the electrode body 102. Alternatively, the distal end 112 of the conductor 110 may be connected to other points along the electrode body 102.

In accordance with the invention, the electrical resistance of the ring electrode adjacent to each of the electrode ends 106 and 108 is greater than that of an intermediate portion 116 of the electrode between the ends. More specifically, adjacent to the distal end 106 of the ring electrode is a region 118; similarly, adjacent to the proximal end 108 is a region 120. The electrical resistance of each of the end regions 118 and 120 is higher than that of the intermediate portion 116 that essentially comprises an exposed portion of the electrode body 102. Accordingly, the higher resistance end regions reduce electrical current flowing through them. In the specific example of the ring electrode illustrated in **FIG. 5****,** the end regions 118 and 120 comprise thin, tapered coatings 122 and 124, respectively, on the outer surface 104 of the electrode body 102. By way of example and not limitation, the length of each of the coatings 122 and 124 may be about 1 mm. Thus, the distal end coating 122 has a proximal extremity 126 of essentially zero thickness and a distal extremity 128, preferably lying in the transverse plane of the electrode end 106, of approximately 0.1 mm in thickness; the outer surface 130 of the coating 122 may comprise a substantially linear variation in thickness between the extremities 126 and 128 although it will be evident that other variations may be utilized. The thin, tapered coatings 122 and 124 increase the electrical resistance of the electrode going toward the respective ends 106 and 108 of the ring electrode. In this particular example, the surface resistivity of the ring electrode varies from essentially 0 ohm-cm² at the proximal extremity 126 of the coating 122 to about 1,000 ohm-cm² at the distal extremity 128. Thus, a preferred way to achieve an electrical resistance that increases towards the electrode ends 106 and 108 is to use an increasing thickness of a coating material of constant resistivity. Each of the coatings 122 and 124 may comprise any material that offers moderately high levels of surface resistivity of, for example, 100 to 5,000 ohm-cm². By way of example only, each of the coatings may comprise parylene, Gortex, silicone rubber, polyethylene, PTFE, ePTFE, ETFE, FEP, PVDF, epoxy, PEEK, polysulfone, or polyurethane lightly doped with a conductive filler. By way of example, the conductive filler may comprise titanium, titanium nitride, ruthenium, silver, stainless steel, iridium, iridium oxide, silver-coated nickel, silver-coated glass, carbon black, graphite, tantalum, palladium, titanium, platinum, gold, MP35N, fullerines, carbon nanotubes, alloys of any of the aforementioned materials, and appropriately sized particles of the conductive polymers polyacetylene, polypyrrole, polyaniline, polythiophene, fluorophenyl thiophene, polyphenylene vinylene, polyphenylene sulfide, polynaphthalene and polyphenylene. In accordance with one preferred form of the invention, the coating may comprise a patterned coating of parylene in which microscopic holes are left in the parylene. In the latter case, a tapered profile may be achieved by decreasing the size and density of the holes in the parylene coating near the electrode ends 106 and 108.

The proximal coating 124 may be substantially the mirror image of the distal coating 122, being placed symmetrically of the transverse, central plane 100.

The extremities of the annular body 102 at the ends 106 and 108 may also be coated but this is not necessary because the annular body extremities tend not to come into contact with the heart tissue.

The upper plot in **FIG. 5** shows the variation in surface resistivity with distance along the length of the electrode from the center of the electrode to each of the electrode ends 106 and 108. The surface resistivity in this example increases from substantially 0 ohm-cm² along the intermediate portion 116 to a maximum of 1,000 ohm-cm² at the ends. The current density plot shows that the current density is relatively constant with local maxima at the ends and in the middle of the ring electrode. The dramatic reduction of the current density at the ends of the electrode increases the magnitude of the current that must be delivered to the ring electrode in order for it to pace anodally, thereby inhibiting the tendency to so pace.

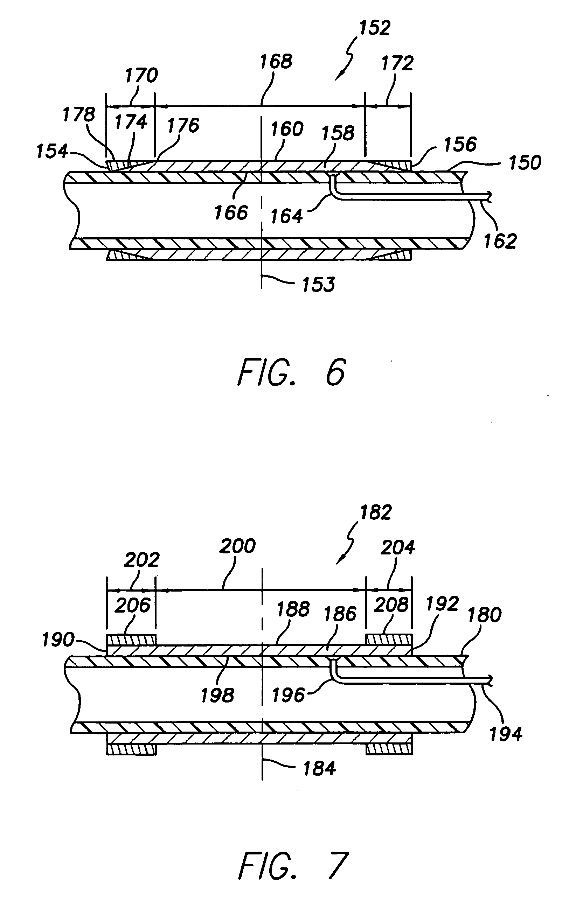

Another specific, exemplary embodiment of the present invention is shown in **FIG. 6. FIG. 6** shows a portion of the lead body of a bipolar transvenous lead, such as that shown in **FIG. 4****,** comprising an insulating, polymer tubular housing 150 carrying a ring electrode 152 having opposed distal and proximal ends 154 and 156, respectively, positioned symmetrically about a central transverse plane 157, the electrode 152 further comprising a main, annular body 158 having an outer cylindrical surface 160. By way of example only, the ring electrode body 158 may have a length, L, of 1.0 cm, a radius, R, of 0.15 cm, and a thickness, T, of 0.1 mm. The electrode body 158 may be fabricated of, for example, MP35N alloy, a platinum/iridium alloy, stainless steel, or titanium, preferably coated with an agent such as titanium nitride, iridium oxide, platinum black, or the like.

The ring electrode 152 is connected to a terminal contact on the connector assembly of the lead by means of an electrical conductor 162 having a distal extremity 164 electrically connected, for example, by a laser weld or by crimping, to a central portion of an inner surface 166 of the electrode body 158. Other connection points along the electrode body 158 may be used.

In accordance with the invention, the electrical resistance of the ring electrode 152 adjacent to each of the electrode ends 154 and 156 is greater than that of an intermediate portion 168 of the electrode between the ends. More specifically, adjacent to the distal end 154 of the ring electrode is a region 170; similarly, adjacent to the proximal end 156 is a region 172. The electrical resistance of each of the end regions 170 and 172 is higher than that of the intermediate portion 168 essentially comprising an exposed portion of the electrode body 158. Accordingly, the higher resistance end regions reduce electrical current flowing through them. Using the end region 170 as representative, the end region 170 is formed by machining, crimping, swaging or otherwise relieving the corresponding end of the electrode body 158 so as to define a repository 174 preferably varying in thickness from the full thickness of the ring electrode body at the end 154 to substantially zero thickness at a proximal extremity 176. The repository 174 is filled with a resistance material 178 such as those previously described, for example, an electrically conductive polymer such as carbon-doped silicone, preferably trimmed so as to be substantially flush with the outer surface 160 of the electrode body 158. As a result of the change in depth of the electrically conductive polymer as a function of distance along the length of the polymer, the surface resistivity will vary, for example, from near zero ohm-cm² at the extremity 176 to, for example, several hundred ohm-cm² at the end 154 of the electrode. The ring electrode 152 will exhibit a current density vs. distance characteristic along the lines of that shown in the current density plot in **FIG. 5****.**

By way of example and not limitation, the length of each of the regions 170 and 172 may range, for example, from 0.1 mm to 3 mm, with a preferred length being 1 mm, with a substantially linear variation in thickness between the extremities 154 and 176 although it will be evident that non-linear variations may be utilized. The varying thickness filling 178 increases the electrical resistance of the region 170 going toward the respective end 154 of the ring electrode.

The proximal region 172 is preferably the substantial mirror image of the distal region 170.

**FIG. 7** shows another specific, exemplary embodiment of the present invention. **FIG. 7** shows a portion of the lead body of a bipolar, transvenous pacing lead such as that of **FIG. 4****,** comprising an insulating, polymer housing 180 carrying a ring electrode 182 designed to inhibit the tendency of the ring electrode to pace anodally. More specifically, the ring electrode 182, preferably formed symmetrically about a central, transverse plane 184, has a main, annular body 186 having an outer cylindrical surface 188 and opposed distal and proximal ends 190 and 192, respectively. By way of example only, the ring electrode body 186 may have a length, L, of 1.0 cm, a radius, R, of 0.15 cm, and a thickness, T, of 0.1 mm. The electrode body 186 may be fabricated of, for example, MP35N alloy, a platinum/iridium alloy, stainless steel, or titanium, preferably coated with an agent such as titanium nitride, iridium oxide, platinum black, or the like.

The ring electrode 182 is connected to a terminal contact on the lead's connector assembly by means of an electrical conductor 194 having a distal extremity 196 electrically connected, for example, by a laser weld or by crimping, to a central portion of an inner surface 198 of the electrode body 186. Other connection points along the electrode body may be used.

In accordance with one aspect of the invention, the electrical resistance of the ring electrode adjacent to each of the electrode ends 190 and 192 is greater than that of an intermediate portion 200 of the electrode between the ends. More specifically, adjacent to the distal end 190 of the ring electrode is a region 202; similarly, adjacent to the proximal end 192 is a region 204. The electrical resistance of each of the end regions 202 and 204 is higher than that of the intermediate portion 200 essentially comprising an exposed portion of the electrode body 186. Accordingly, the higher resistance end regions reduce electrical current flowing through them. In the specific example of the ring electrode illustrated in **FIG. 7****,** the end regions 202 and 204 comprise substantially constant thickness layers or coatings 206 and 208, respectively, deposited on the outer surface 188 of the electrode body 186. By way of example and not limitation, the length of each of the coatings 206 and 208 may be about 1 mm, and the thickness of each of the coatings may be about 0.2 mm. The resistivity of each of the coatings may range, by way of example, from 0.01 to 1,000 ohm-cm. The coating material may comprise any of the materials previously described herein, for example, an electrically conductive polymer such as silicone rubber lightly doped with carbon. Generally, each of the coatings may comprise any material that offers moderately high levels of resistance.

Although not providing electrical resistances that vary along their lengths, the constant thickness coatings 206 and 208 work sufficiently well to mitigate the "hot spot" problem and the current density variation will resemble the plot shown in **FIG. 5****.**

The extremities of the annular body 186 at the ends 190 and 192 may also be coated but this is not necessary because the extremities tend not to come into contact with the heart tissue.

**FIG. 8** shows a multipolar, transvenous pacing, sensing and defibrillating system 220 in accordance with an alternative embodiment of the invention. The system 220 comprises a lead 222 and an implantable medical device (IMD) 224 that may comprise a pacemaker/ICD. The lead 220 includes a lead body 226 having a proximal end 228 and a distal end portion 230. The proximal end 228 of the lead body 226 incorporates a coaxial connector assembly 232 that may be compatible with a standard such as the proposed IS-4 standard for connecting the lead body 226 to the IMD 224. In the example shown in **FIG. 8****,** the connector assembly 232 includes a tubular pin terminal contact 234 and three annular terminal contacts 236, 238 and 240 electrically coupled to electrodes along the distal end portion of the lead body. The connector assembly 232 is received within a receptacle (not shown) in the IMD 224 containing electrical terminals positioned to engage the terminal contacts 234, 236, 238 and 240 on the connector assembly. The lead body 226 preferably comprises a tubular sheath or housing 242 made of an insulating, biocompatible, biostable polymer, as described earlier.

In the embodiment of **FIG. 8****,** the distal end portion 230 of the lead body carries a tip electrode 244 and two ring electrodes 246 and 248 proximally of the tip electrode. The tip and ring electrodes are coupled to corresponding terminal contacts 234, 236 and 238, respectively, on the connector assembly 232 by means of electrical conductors (not shown) within the housing 242. The distal end portion of the lead body also carries a cardioverting and/or defibrillating electrode 250 electrically connected to the terminal contact 240 by means of a separate electrical conductor (not shown) within the housing 242.

In conventional fashion, the distal end portion of the lead body 226 may include passive and/or active fixation or anchoring means 252 of the kind already described

Other electrode arrangements may be employed pursuant to lead constructions well known in the art. For example, an alternative electrode arrangement may include additional ring stimulation and/or sensing electrodes as well as additional cardioverting and/or defibrillating coils spaced apart along the distal end of the lead body. Thus, **FIG. 8** is illustrative only, depicting one example of a multipolar pacing lead comprising at least two ring electrodes. In accordance with the present invention, each of the electrodes 246 and 248 comprises a ring electrode of the kind described above in connection with the examples of **FIGS. 4** through 7. Accordingly, as already explained, each of the ring electrodes 246 and 248 may have end regions characterized by electrical resistances greater than that of the portion of the ring electrode between the end regions so that during operation of the lead 220, the tendency for these electrodes to anodally pace is substantially eliminated.

Since, in accordance with the invention, there are no edges of the ring electrode to concentrate the electrical current, the tendency for anodal ring pacing to occur is substantially eliminated. Accordingly, pacing will occur at the tip and full bipolar automatic capture pacing may be realized.

It will be evident that many variations of leads in accordance with the teaching of the invention are made possible for both right side and left side heart stimulation and sensing or combinations thereof, and the ring electrode configurations shown in the various drawing figures are examples only, and are not intended to be exhaustive.

## Claims

1. An implantable pacing lead (62) comprising: a lead body (66) defining a proximal end (68) and a distal end (70) portion and comprising a connector assembly (72) at the proximal end (68); and a ring electrode (92) positioned along the lead body (66), the ring electrode (92) being electrically coupled to the connector assembly (72); in which the ring electrode (92) defines distal (106) and proximal (108) ends, and the electrical resistance of the ring electrode (92) adjacent each of the ends (106,108) is greater than that of the portion (116) of the ring electrode (92) between the ends (106,108), **characterised in that** said ring electrode (92) consists of a single annular electrode body having an annular end region (118, 120) adjacent each of the ends (106, 108) of the ring electrode (92), and each of the annular end regions comprises a coating on the outer surface of the electrode body.

2. A lead as claimed in Claim 1, **characterised in that** it further includes: a tip electrode (90) at a distal extremity of the distal end portion (70) of the lead body (66), the tip electrode (90) being electrically coupled to a first terminal contact (74) on the connector assembly (72) attached to the proximal end (68) of the lead body (66); and the ring electrode (92) is positioned along the distal end portion (70) of the lead body (66) proximally of the tip electrode (90), the ring electrode (92) being electrically coupled to a second terminal contact (76) on the connector assembly (72),

3. A lead as claimed in Claim 1, **characterised in that** the lead has at least two ring electrodes positioned along the lead body, each of the at least two ring electrodes being electrically coupled to a corresponding terminal contact on the connector assembly, each of the at least two ring electrode having a distal end and a proximal end, each of the end regions having an electrical resistance greater than that of the portion of the respective ring electrode between the end regions, whereby, the lead constitutes a multipolar pacing lead.

4. A lead as claimed in any of Claims 1 to 3, **characterised in that** the electrical resistance of each of the end regions (202, 204) is substantially constant along the length of the region.

5. A lead as claimed in any of Claims 1 to 3, **characterised in that** each of the end regions extends outwardly along the length of the or each ring electrode (92) from an inner extremity of the region, and the electrical resistance of each of the end regions (118, 120; 170, 172) increases outwardly from the inner extremity of the end region.

6. A lead as claimed in Claim 5, **characterised in that** the coating at the ends of the or each ring electrode has an increasing thickness of a coating material of constant resistivity, thereby achieving an electrical resistance that increases towards the electrode ends.

7. A lead as claimed in Claim 5, **characterised in that** the or each ring electrode comprises an annular electrode body (160) having a reduced thickness portion within each of the annular end regions (170, 172), each of the reduced thickness portions comprising a repository (174) filled with an electrically conductive, relatively high electrical resistance material.

8. A lead as claimed in any preceding claim, **characterised in that** each of the coatings comprises a material selected from parylene, Gortex, silicone rubber, polyethylene, PTFE, ePTFE, ETFE, FEP, PVDF, epoxy, PEEK, polysulfone, or polyurethane lightly doped with a conductive filler.

9. A lead as claimed in Claim 8, **characterised in that** the conductive filler comprises a material selected from the group consisting of titanium, titanium nitride, ruthenium, silver, stainless steel, iridium, iridium oxide, silver-coated nickel, silver-coated glass, carbon black, graphite, tantalum, palladium, titanium, platinum, gold, MP35N, fullerines, carbon nanotubes, alloys of any of the aforementioned materials, and particles of the conductive polymers polyacetylene, polypyrrole, polyaniline, polythiophene, fluorophenyl thiophene, polyphenylene vinylene, polyphenylene sulfide, polynaphthalene and polyphenylene.

10. A lead as claimed in any of Claims 3 to 9, **characterised in that** each of the coatings has a length extending along the length of the electrode body from an inner extremity of the coating to an outer extremity of the coating adjacent to a corresponding end of each of the at least two ring electrodes, each of the coatings having a thickness that increases along the length of the coating from the inner extremity of the coating to the outer extremity thereof.

11. A lead as claimed in any of Claims 3 to 9, **characterised in that** each of the coatings has a length extending along the length of the electrode body from an inner extremity of the coating to an outer extremity of the coating adjacent to a corresponding end of each of the at least two ring electrodes, each of the coatings having a thickness that is substantially constant along the length of the coating.

## Patentansprüche

1. Implantierbare Schrittmacherelektrode (62) umfassend einen Elektrodenkörper (66), der einen proximalen Endabschnitt (68) und einen distalen Endabschnitt (70) definiert und eine Steckverbinderanordnung (72) am proximalen Ende (68) aufweist, und eine Ringelektrode (92), die am Elektrodenkörper (66) angebracht ist, wobei die Ringelektrode (92) mit der Steckverbinderanordnung (72) elektrisch verbunden ist, wobei die Ringelektrode (92) distale (106) und proximale (108) Enden definiert und wobei der elektrische Widerstand der Ringelektrode (92) nahe der Enden (106, 108) größer ist als der im Bereich (116) der Ringelektrode (92) zwischen den Enden (106, 108),
**dadurch gekennzeichnet,**
**dass** die Ringelektrode (92) aus einem einzelnen ringförmigen Elektrodenkörper besteht, der einen ringförmigen Endbereich (118, 120) jeweils benachbart zu den Enden (106, 108) der Ringelektrode (92) aufweist, und jeder der ringförmigen Endbereiche eine Beschichtung auf der Außenoberfläche des Elektrodenkörpers aufweist.

2. Elektrode nach Anspruch 1.
**dadurch gekennzeichnet,**
**dass** sie weiter eine Spitzenelektrode (90) am äußersten distalen Ende des distalen Endabschnitts (70) des Elektrodenkörpers (66) umfasst, wobei die Spitzenelektrode (90) elektrisch mit einem ersten Terminalkontakt (74) der Steckverbinderanordnung (72), die am proximalen Ende (68) des Elektrodenkörpers (66) angeordnet ist, verbunden ist wobei die Ringelektrode (92) am distalen Endabschnitt (70) des Elektrodenkörpers (66) proximal der Spitzenelektrode (90) angeordnet ist und wobei die Ringelektrode (92) elektrisch mit einem zweiten Terminalkontakt (76) der Steckverbinderanordnung (72) verbunden ist.

3. Elektrode nach Anspruch 1,
**dadurch gekennzeichnet,**
**dass** die Elektrode mindestens zwei Ringelektroden aufweist, die am Elektrodenkörper angeordnet sind, wobei jede der mindestens zwei Ringelektroden elektrisch mit einem entsprechenden Terminalkontakt der Steckverbinderanordnung verbunden ist, jede der mindestens zwei Ringelektroden ein distales Ende und ein proximales Ende aufweist und jeder der Endabschnitte einen elektrischen Widerstand aufweist, der größer ist als der des Bereichs der jeweiligen Ringelektrode zwischen den Endbereichen, so dass die Elektrode eine multipolare Schrittmacherelektrode bildet.

4. Elektrode nach einem der Ansprüche 1 bis 3,
**dadurch gekennzeichnet,**
**dass** der elektrische Widerstand jedes der der Endbereiche an den Enden (202. 204) über die Lange des Bereiches im Wesentlichen konstant ist.

5. Elektrode nach einem der Ansprüche 1 bis 3,
**dadurch gekennzeichnet,**
**dass** jeder der Endbereiche sich länge der mindestens einen Ringelektrode (92) von einem inneren Ende des Bereichs nach außen erstreckt und der elektrische Widerstand jedes der Endbereiche (118, 120, 170, 172) von einem inneren Ende des Endbereichs nach außen anwächst.

6. Elektrode nach Anspruch 5,
**dadurch gekennzeichnet,**
**dass** die Schicht an den Enden der mindestens einen Ringelektrode eine zunehmende Dicke eines Beschichtungsmaterials mit einem konstanten elektrischen Widerstand aufweist, wodurch sich der elektrische Widerstand zu den Endbereichen der Elektrode hin erhöht.

7. Elektrode nach Anspruch 5,
**dadurch gekennzeichnet,**
**dass** die mindestens eine Ringelektrode einen ringförmigen Elektrodenkörper (160) aufweist, der einen Abschnitt geringerer Dicke innerhalb jedes der ringförmigen Endbereiche (170, 172) aufweist, wobei jeder Abschnitt geringere Dicke eine Kammer (174) aufweist, die mit einem elektrisch leitenden Material mit verhältnismäßig hohem elektrischen Widerstand gefüllt ist.

8. Elektrode nach einem der vorstehenden Ansprüche,
**dadurch gekennzeichnet,**
**dass** jede der Beschichtungen eines der folgenden Materialien aufweist: Parylen, Gortex, Silikongummi, Polyäthylen, PTFE, ePTFE, ETFE, FEP, PVDF, Epoxid, PEEK, Polysulphon oder Polyurethan, das geringfügig mit einem leitenden Füllmaterial dotiert ist.

9. Elektrode nach Anspruch 8,
**dadurch gekennzeichnet,**
**dass** das leitende Füllmaterial ein Material enthält, das aus der folgenden Gruppe stammt: Titan, Titannitrid, Ruthenium, Silber, rostfreier Stahl, Iridium, Iridiumoxid, silberbeschichtetes Nickel, silberbeschichtetes Glas, Ruß, Graphit, Tantal, Palladium, Titan, Platin, Gold, MP35N, Fullerene, Carbon-Nanotubes. Legierungen beliebiger der vorher erwähnten Materialien und Partikel der leitenden Polymere Polyacetylen, Polypyrrol, Polyanilin, Polythiophen, fluorophenyl Thiophen, Polyphenylen Vinylen, Polyphenylen sulfid, Polynaphthalen und Polyphenylen.

10. Elektrode nach einem der Ansprüche 3 bis 9,
**dadurch gekennzeichnet,**
**dass** jede der Beschichtungen eine Länge aufweist, die sich längs des Elektrodenkörpers von einem inneren Ende der Beschichtung bis zu einem äußeren Ende der Beschichtung benachbart zu einem entsprechenden Ende der mindestens zwei Ringelektroden erstreckt, wobei jede der Beschichtungen eine Dicke aufweist, die längs der Beschichtung vom inneren Ende der Beschichtung bis zu ihrem äußeren Ende anwächst.

11. Elektrode nach einem der Ansprüche 3 bis 9,
**dadurch gekennzeichnet,**
**dass** jede der Beschichtungen eine Länge aufweist, die sich längs des Elektrodenkörpers von einem inneren Ende der Beschichtung bis zu einem äußeren Ende der Beschichtung benachbart zu einem entsprechenden Ende der mindestens zwei Ringelektroden erstreckt, wobei jede der Beschichtungen eine Dicke aufweist, die längs der Beschichtung im Wesentlichen konstant ist.

## Revendications

1. Fil de stimulateur cardiaque implantable (62) comprenant : un corps de fil (66) définissant une extrémité proximale (68) et une partie d'extrémité distale (70) et comprenant un ensemble de connecteur (72) à l'extrémité proximale (68) ; et une électrode annulaire (92) positionnée le long du corps de fil (66), l'électrode annulaire (92) étant couplée électriquement à l'ensemble de connecteur (72); dans lequel l'électrode annulaire (92) définit les extrémités distale (106) et proximale (108), et la résistance électrique de l'électrode annulaire (92) adjacente à chacune des extrémités (106, 108) est supérieure à celle de la partie (118) de l'électrode annulaire (92) entre les extrémités (106, 108), **caractérisé en ce que** ladite électrode (92) est constituée d'un seul corps d'électrode annulaire présentant une zone d'extrémité annulaire (118, 120) adjacente à chacune des extrémités (106, 108) de l'électrode annulaire (92), et chacune des zones d'extrémité annulaire comprend un revêtement sur la surface externe du corps électrique.

2. Fil selon la revendication 1, **caractérisé en ce qu'**il comporte en outre : une électrode à pointe (90) à une extrémité distale de la partie d'extrémité distale (70) du corps de fil (66), l'électrode à pointe (90) étant couplée électriquement à un premier contact de borne (74) sur l'ensemble de connecteur (72) fixé à l'extrémité proximale (68) du corps de fil (66); et l'électrode annulaire (92) est positionnée le long de la partie d'extrémité distale (70) du corps de fil (66) de manière proximale par rapport à l'électrode à pointe (90), l'électrode annulaire (92) étant couplée électriquement à un second contact de borne (76) sur l'ensemble de connecteur (72).

3. Fil selon la revendication 1, **caractérisé en ce que** le fil présente au moins deux électrodes annulaires positionnées le long du corps de fil, chacune de les au moins deux électrodes annulaires étant couplée électriquement à un contact de borne correspondant sur l'ensemble de connecteur, chacune de les au moins deux électrodes annulaires présentant une extrémité distale et une extrémité proximale, chacune des zones d'extrémité présentant une résistance électrique supérieure à celle de la partie de l'électrode annulaire respective entre les zones d'extrémité, le fil constituant un fil de stimulation cardiaque multipolaire.

4. Fil selon l'une quelconque des revendications 1 à 3, **caractérisé en ce que** la résistance électrique de chacune des zones d'extrémité (202, 204) est sensiblement constante le long de la longueur de la zone.

5. Fil selon l'une quelconque des revendications 1 à 3, **caractérisé en ce que** chacune des zones d'extrémité s'étend vers l'extérieur le long de la longueur de la ou chaque électrode annulaire (92) à partir d'une extrémité interne de la zone, et la résistance électrique de chacune des zones d'extrémité (118, 120 ; 170, 172) augmente vers l'extérieur à partir de l'extrémité interne de la zone d'extrémité.

6. Fil selon la revendication 5, **caractérisé en ce que** le revêtement aux extrémités de la ou chaque électrode annulaire présente une épaisseur croissante d'un matériau de revêtement d'une résistivité constante, obtenant de ce fait une résistance électrique qui augmente en direction des extrémités de l'électrode.

7. Fil selon la revendication 5, **caractérisé en ce que** la ou chaque électrode annulaire comprend un corps d'électrode annulaire (160) présentant une partie d'épaisseur réduite à l'intérieur de chacune des zones d'extrémité annulaire (170, 172), chacune des parties d'épaisseur réduite comprenant un dépôt (174) rempli d'un matériau électriquement conducteur, d'une résistance électrique relativement élevée

8. Fil selon l'une quelconque des revendications précédentes, **caractérisé en ce que** chacun des revêtements comprend un matériau sélectionné parmi le parylène, le gortex, le caoutchouc siliconé, le polyéthylène, le PTFE, l'ePTFE, l'ETFE, le FEP, le PVDF, la résine époxy, le PEEK, la polysulfone, ou le polyuréthane légèrement dopé avec une charge conductrice.

9. Fil selon la revendication 8, **caractérisé en ce que** la charge conductrice comprend un matériau sélectionné parmi le groupe constitué du titane, du nitrure de titane, du ruthénium, de l'argent, de l'acier inoxydable, de l'iridium, de l'oxyde d'iridium, du nickel revêtu d'argent, du verre revêtu d'argent, du noir de carbone, du graphite, du tantale, du palladium, du titane, du platine, de l'or, du MP35N, des fullerines, des nanotubes de carbone, des alliages de n'importe quel parmi les matériaux mentionnés ci-dessus, et des particules des polymères conducteurs polyacéthylène, polypyrrole, polyaniline, polythiophène, fluorophényle thiophéne, polyphénylène vinylène, polyphénylène sulfide, polynaphtalène et polyphénylène.

10. Fil selon l'une quelconque des revendications 3 à 9, **caractérisé en ce que** chacun des revêtements présente une longueur s'étendant le long de la longueur du corps d'électrode d'une extrémité interne du revêtement à une extrémité externe du revêtement adjacente à une extrémité correspondante de chacune de les au moins deux électrodes annulaires, chacun des revêtements présentant une épaisseur qui augmente le long de la longueur du revêtement de l'extrémité interne du revêtement à son extrémité externe.

11. Fil selon l'une quelconque des revendications 3 à 9, **caractérisé en ce que** chacun des revêtements présente une longueur s'étendant le long de la longueur du corps d'électrode d'une extrémité interne du revêtement à une extrémité externe du revêtement adjacente à une extrémité correspondante de chacun des au moins deux électrodes annulaires, chacun des revêtements présentant une épaisseur qui est sensiblement constante le long de la longueur du revêtement.
